# EUROPÄISCHE PATENTSCHRIFT

(11) **EP 1 439 762 B1**
(45) Veröffentlichungstag und Bekanntmachung des Hinweises auf die Patenterteilung: **09.08.2006**
(21) Anmeldenummer: 02769833.1
(22) Anmeldetag: 01.11.2002
(51) Int. Cl.: A23L 3/00, A23L 3/10, A23L 3/12, A61L 2/07, A61L 2/00, A61L 2/24, B65B 25/00

(54) **VERFAHREN ZUR ENTKEIMUNG VON PRODUKTEN**
METHOD FOR STERILISING PRODUCTS
PROCEDE POUR STERILISER DES PRODUITS

(30) Priorität: 02.11.2001 CH 200701
(43) Veröffentlichungstag der Anmeldung: 28.07.2004
(73) Patentinhaber: STEAMLAB SYSTEMS AG NATURAL PASTEURIZATION, CH-4103 Bottmingen (CH)
(72) Erfinder: BLAHA, Jirka, 79539 Lörrach (DE)
(74) Vertreter: Luchs, Willi
(86) Internationale Anmeldenummer: PCT/CH2002/000591
(87) Internationale Veröffentlichungsnummer: WO 2003/037109

(56) Entgegenhaltungen:
- EP-A- 0 532 839
- EP-A- 0 891 714
- EP-A- 0 998 855
- DE-C- 10 028 183
- FR-A- 2 768 594

## Beschreibung

Die Erfindung betrifft ein Verfahren zur Entkeimung von Produkten, insbesondere von Nahrungsmitteln und deren Rohstoffen, Hilfsstoffen und Halbfabrikaten, sowie von Roh-, Hilfs- und Zwischenstoffen für die pharmazeutische und kosmetische Industrie, gemäss dem Oberbegriff des Anspruches 1 sowie eine Vorrichtung zur Durchführung des Verfahrens.

Es sind verschiedene Verfahren und Anlagen zur natürlichen Entkeimung von pflanzlichen, tierischen oder künstlichen Materialien bekannt, bei denen das zu entkeimende Gut, vorzugsweise in Säcke abgefüllt, in einer verschliessbaren, evakuierbaren Behandlungskammer unter Einsatz von Dampf bzw. Sattdampf der Entkeimung unterzogen wird.

Aus der EP-A-0 998 855 ist auch bekannt, dass in die Behandlungskammer ein oder mehrere Beschickungsbehälter mit dem zu entkeimenden Gut eingebracht werden können, wodurch insbesondere die Entkeimung von pulverformigen Materialien verbessert wird (kürzere Behandlungszeit, gleichmässigere Behandlung des zu entkeimenden Gutes).

Die Druckschrift EP-A-0 532 839 beschreibt ein Verfahren zum Entkeimen eines schüttbaren Produktes, bei dem nach dem Schliessen eines Behälters als erster Verfahrensschritt ein konstanter Unterdruck im Innenraum erzeugt wird. Sodann wird das entsprechende Ventil geschlossen und Dampf mit einem vorgegebenen Druck eingeblasen. Der Dampfdruck soll zwischen 1,5 bis 7 bar und die gewählte Temperatur zwischen 110 bis 165°C betragen (siehe Spalte 6, Zeilen 3 bis 8). In einem nächsten Verfahrensschritt wird der Dampf aus dem Innenraum entfernt und zu einem Kondensator geführt. Im Anschluss daran wird das Produkt gekühlt, indem erneut ein Unterdruck erzeugt wird. Abschliessend wird dieser Unterdruck abgebaut und in der Folge kann der Behälter wieder geöffnet werden. Diese Verfahrensschritte sind insbesondere in den Spalten 5, Zeile 43 bis Spalte 6, Zeile 30 erläutert.

Bei diesem Verfahren sind verschiedene Nachteile vorhanden. Beim ersten Verfahrensschritt, bei dem ein konstanter, einmalig erzeugter Unterdruck im geschlossenen Innenraum des Behälters erzeugt wird, besteht die Gefahr, dass sich dieser Unterdruck nicht überall, insbesondere in der zu sterilisierenden Schicht des Gutes nicht ausreichend ausbreitet. Dies bewirkt, dass der nachfolgend in den Behälter eingeführte Dampf nicht schnell genug mit dem zu entkeimenden Gut vollumfänglich in Kontakt tritt und dass daher eine homogene und gleichsam gänzliche Entkeimung des gesamten Gutes nicht wirklich stattfindet. Zudem bewirken der hohe Dampfdruck von mindestens 1,5 bar sowie die hohen Temperaturen von 110°C bis 165°C, dass eine schonende und homogene Entkeimung nicht gegeben ist und die sensorischen Eigenschaften, wie Aroma, Farbe, Geschmack und anderes, verloren gehen, als auch die ernährungsphysiologischen Werte, zum Beispiel Vitamine, Mineralstoffe, Kohlenhydrate etc. zumindest teilweise vernichtet werden.

Bei der Druckschrift EP-A-891 714 ist ein Verfahren zum Entkeimen von Einzelportionen, beispielsweise von Gemüse oder Früchten beschrieben, bei welchem jeweils ein Behältnis mit dem Keimgut in eine Druckkammer gestellt wird. In diese geschlossene Druckkammer wird dann Dampf eingeblasen und dies bei einer gewünschten Temperatur und einer gewissen Zeitspanne. Im Anschluss daran wird diese Dampfzufuhr unterbrochen und Sterilluft, Schutzgas oder ein Kühlwasser zur Aufrechterhaltung des Druckes in die Druckkammer gebracht.

Der vorliegenden Erfindung liegt die Aufgabe zugrunde, ein Verfahren der eingangs genannten Art und eine Vorrichtung zum Durchführen dieses Verfahrens zu schaffen, welche eine besonders effiziente und schonende Entkeimung und ggf. Entwesung unter Beibehaltung homogener und weitgehend unveränderter Produkteigenschaften (Qualität) ermöglichen.

Diese Aufgabe wird erfindungsgemäss durch ein Verfahren mit den Merkmalen des Anspruches 1 sowie durch eine Vorrichtung mit den Merkmalen des Anspruches 14 gelöst.

Bevorzugte Weitergestaltungen der Erfindung bilden den Gegenstand abhängiger Ansprüche.

Das erfindungsgemässe Verfahren sowie die erfindungsgemässe Vorrichtung zum Durchführen desselben ermöglichen eine konstante und reproduzierbare Behandlung der Produkte. Daraus resultiert ein weitgehender Erhalt der Homogenität, der sensorischen Eigenschaften (Aroma, Farbe, Geschmack, Körnigkeit, usw.), der funktionellen Inhaltsstoffe und der ernährungsphysiologischen Werte (Vitamine, Mineralstoffe, Kohlenhydrate, Fette, Eiweiss usw.) der Produkte.

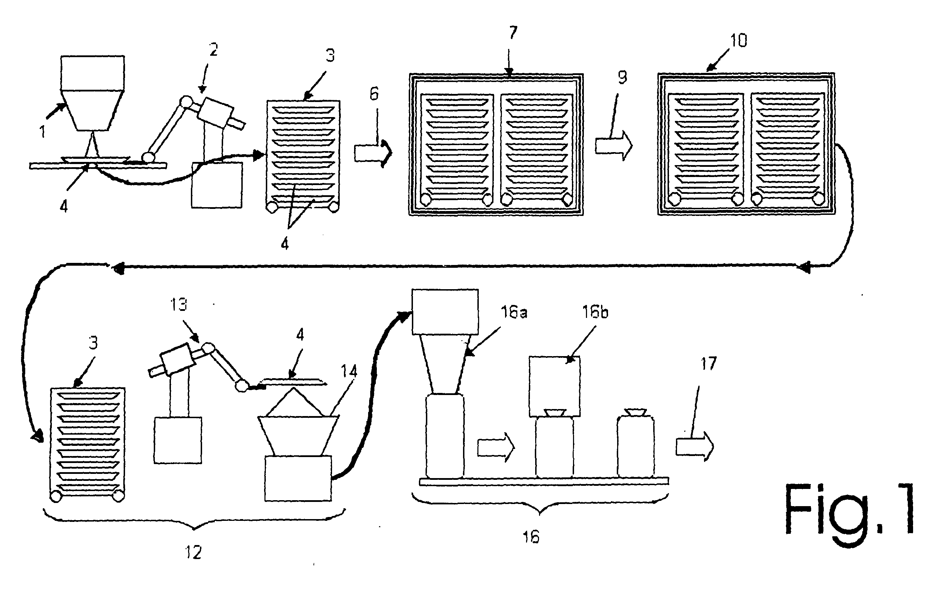

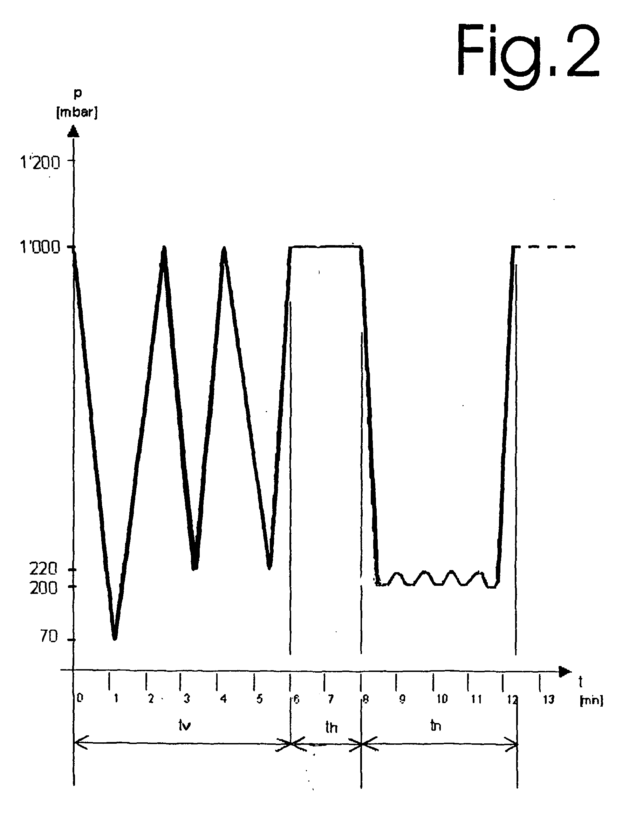

Die Erfindung wird nachfolgend anhand von Fig. 1 und 2 näher erläutert.
- Fig. 1: Schematische Darstellung eines erfindüngsgemässen Verfahrens und einer erfindungsgemässen Vorrichtung; und
- Fig. 2: Druck/Zeit-Diagramm eines erfindungsgemässen Entkeimungs-und Entwesungsprozesses.

Fig. 1 zeigt schematisch ein Verfahren und eine Vorrichtung zur Entkeimung und Entwesung. Diese sind für die Behandlung von Produkten, insbesondere Trockenprodukten, wie Nahrungsmitteln und deren Rohstoffen, Hilfsstoffen und Halbfabrikate, sowie von Roh-, Hilfs- und Zwischenstoffen für die pharmazeutische und kosmetische Industrie geeignet. Die zu behandelnden Produkte werden in Pulverform, als Granulate, als Flocken sowie in ganzen oder geschnittenen Stücken (z.B. Kerne, Nüsse, Blätter, Wurzeln, getrocknetes Obst und Gemüse usw.) angeliefert. Als Transportbehälter kommen verschiedene Gebinde bzw. Behälter (z.B. Schachteln, sogenannten Big Bags, Säcke, Silos usw.) in Betracht. Die Produkte werden jeweils einer Eingangskontrolle unterzogen. Anhand eines Referenzmusters werden die Produkte auf Wassergehalt, sensorische Eigenschaften (z.B. Aussehen, Geruch, Aroma, Geschmack usw.) und weitere produktspezifische Eigenschaften geprüft. Die Resultate werden protokollarisch festgehalten. Bis zum Beginn der Behandlung werden die Produkte sortengetrennt gelagert. Dabei werden besondere Lagerungs-Vorschriften und Richtlinien hinsichtlich einer allfälligen Geruchsübertragung sowie bezüglich der Richtlinien für die Behandlung von Bio-Produkten berücksichtigt. Die Lagerung gewisser Produkte erfolgt in gekühlter Umgebung.

Die zu behandelnden Produkte werden unmittelbar vor der Behandlung erneut auf ihren Wassergehalt geprüft. Vor der Behandlung in der Druck- bzw. Entkeimungskammer werden gewisse Produkte eventuell einer oder mehreren mechanischen Behandlungen unterzogen (es kann sich z.B. um das Auflockern von klebrigen Produkten oder um das Sieben vom verklumpten Produkten handeln).

Der eigentliche Behandlungsprozess beginnt mit dem Einfüllen der Produkte (über einen Trichter) in eine Dosiervorrichtung 1. Mit Hilfe der Dosiervorrichtung 1 werden die Produkte nach Bedarf in definierten Schichtdicken auf Tablare 4 geschichtet. Insbesondere bei pulver-, granulat- oder flockenförmigen Produkten wird die Schichtdicke exakt eingehalten. Die Tablare 4 mit der zu behandelnden Ware werden manuell oder automatisch in ein als Tablarträger 3 gestaltetes Prozessgebinde eingebracht. Anstelle der Tablarträger 3 und der Tablare 4 können selbstverständlich auch andere Prozessgebinde bzw. Beschickungsbehälter Anwendung finden. Das Prozessgebinde kann auch als Sackträger ausgebildet sein, welcher die gleichmässige Bedampfung der Sackoberflächen sicherstellt.

Wie in Fig. 1 mit Pfeil 6 angedeutet, werden sodann erfindungsgemäss der oder die vorzugsweise fahrbaren Tablarträger 3 manuell oder automatisch durch eine Eingangstür in die Entkeimungskammer 7 eingeschoben.

In der Entkeimungskammer 7 findet der eigentliche, programmgesteuerte Entkeimungsprozess statt. Dieser besteht im wesentlichen aus drei Phasen, nämlich
(a) der Konditionierung,
(b) der Entkeimung und
(c) der Trocknung der Produkte.

Die gasdichte Entkeimungskammer 7 ist in nicht näher dargestellter Weise an eine Vakuumpumpe, eine Dampfquelle (vorzugsweise überhitzter Dampf oder Sattdampf) und an eine Luftquelle angeschlossen.

Ein Beispiel, wie ein mit Sattdampf durchgeführter Entkeimungsprozess in besonders vorteilhafter Weise verlaufen kann, zeigt das in Fig. 2 dargestellte Druck/Zeit-Diagramm. Aus diesem ist ersichtlich, dass in einer ersten Phase während einer produktspezifischen Zeitdauer tᵥ ein Gasaustausch und die Erhitzung des zu entkeimenden Gutes durchgeführt wird. Diese sogenannte Konditionierungs-Phase, die in mehreren Vakuumzyklen verläuft, ermöglicht, dass die nachfolgende, sogenannte Behandlungsphase kurz (Zeitdauer tₕ) und bei relativ niedrigen Temperaturen (Sattdampftemperatur von 70°-120°C) erfolgen kann. Zu Beginn der Behandlungsphase ist die Entkeimungskammer 7 unter tiefem Vakuum, womit der schnelle und vollständige Kontakt des Dampfes zu jedem Teil der Oberfläche des Produktes sichergestellt wird. Die absoluten Druckwerte betragen vorzugsweise 50 bis 1000 mbar. Bei gewissen Produkten kann die Behandlungsdauer bei mehr als 100°C noch verkürzt werden (vgl. die gestrichelte Linie in Fig. 2, Zeitdauer tₕ'). Die sogenannte Haltezeit, während der ein konstantes Temperatur- und Druckniveau herrscht, beträgt vorzugsweise 30 s bis 300 s. Unter diesen Bedingungen ist eine schonende, hochwertige Entkeimung, weitgehend ohne Beeinträchtigung der ernährungsphysiologischen und/oderder sensorischen Eigenschaften, gewährleistet. Auf die Behandlungsphase folgt die sogenannte Trocknungsphase, während der das zu entkeimende Gut während einer produktspezifischen Zeitdauer tₙ getrocknet und teilweise abgekühlt wird. Durch eine Druckabsenkung in den Vakuumbereich wird allfälliges Kondenswasser wieder entfernt.

Wie bereits erwähnt verläuft der Entkeimungsprozess in der Entkeimungskammer 7 vollautomatisch und computergesteuert. Der wesentliche Prozessparameter während der Konditionierungsphase ist der Druckwechsel (Druck, Anzahl und Dauer der Vakuumzyklen, Temperatur sowie Geschwindigkeit des Gasaustausches und der Druckabsenkung). Während der Behandlungsphase, dem eigentlichen Entkeimungsprozess, sind es die Faktoren Haltedauer, Druck und Temperatur. Bei der Nachbehandlung bzw. Trocknung sind es Dauer, Druckverlauf, Anzahl der Luftstösse, Luftqualität, sowie Anfangs- und Endtemperatur.

Nach der Beendigung des Entkeimungsprozesses werden der oder die Tablarträger 3 mit den Produkten aus der Entkeimungskammer 7 entnommen. Dies geschieht z.B. durch eine der Eingangstüre gegenüberliegende Ausgangstüre. Die Ware wird dann in Pfeilrichtung 9 in eine Kühlkammer 10 eingeführt. Zu diesem Zeitpunkt beträgt die Temperatur an der Produktoberfläche in der Regel 50 bis 60°C. Die Luft in der Kühlkammer 10 wird bezüglich Temperatur, Feuchtigkeit und Reinheit konditioniert. Für die Kühlbedingungen werden produktspezifische Kriterien vorgegeben. Die wesentlichen Prozessparameter der ebenfalls programmgesteuerten Kühlphase sind die Anfangs- und Endtemperatur sowie die Dauer des Kühlprozesses.

Die Tablare 4 mit den auf die vorgegebene Temperatur gekühlten Produkten werden in einer Entleerungsstation 12 manuell oder automatisch in einem Manipulator 13 oder dergleichen aus den Tablarträgern 3 entnommen und in einen Entleertrichter 14 entleert.

Produkte, die während des Entkeimungprozesses verklumpen oder aneinander haften, werden vor und/oder nach der Kühlung mechanisch behandelt (gemahlen, granuliert, aufgelockert), um wieder die Ausgangskonsistenz zu erreichen. Als Auflockerungsgeräte können Rüttelsieb, Nibbler oder andere Gerätschaften verwendet werden. Wenn die Produkte vor der Kühlung mechanisch behandelt werden, erfolgt die Kühlung in einem dazu geeigneten Prozessgebinde.

Mittels einer Verpackungseinrichtung 16 werden die Produkte sodann in die produktspezifisch vorgegebenen Auslieferungsgebinde bzw. Auslieferungsbehälter abgefüllt (vgl. Bezugszeichen 16a). Diese Gebinde können so mannigfaltig wie die Anlieferungsgebinde ausgebildet sein. Unmittelbar vor dem Verschliessen dieser Gebinde (vgl. Bezugszeichen 16b) wird ein Referenzmuster entnommen.

Die Prozess-Schritte 9 - 16 erfolgen vorzugsweise in Räumlichkeiten, deren Luft hinsichtlich Temperatur, relativer Luftfeuchtigkeit und mikrobieller Belastung konditioniert wird (sogenannter Reinraum).

Mit dem Verschliessen und Wegtransportieren der Auslieferungsgebinde ist der Behandlungsprozess abgeschlossen (mit Pfeil 17 ist das Ausschleusen aus dem Reinraum angedeutet).

Gegebenenfalls könnte das Prozessgebinde auch so gestaltet sein, dass es geeignet ist, die behandelten Produkte in einen weiteren Produktveredelungsprozess einzubringen.

Das vorstehend beschriebene, erfindungsgemässe Verfahren ermöglicht eine effiziente und schonende Entkeimung von Produkten, insbesondere Trockenprodukten, bei der ein weitgehender Erhalt der Homogenität, der sensorischen Eigenschaften (Aroma, Farbe, Geschmack, Körnigkeit usw.), der funktionellen Inhaltsstoffe sowie der ernährungsphysiologischen Werte (Vitamine, Mineralstoffe, Kohlenhydrate, Fette, Eiweiss usw.) der Produkte gewährleistet wird. Ein wesentlicher Vorteil besteht darin, dass die Behandlung reproduzierbar ist und zu homogener Qualität der gesamten Produktecharge führt.

## Patentansprüche

1. Verfahren zur Entkeimung von schuttfähigen Feststoffprokukten insbesondere von Nahrungsmitteln und deren Rohstoffen, Hilfsstoffen und Halbfabrikaten, sowie von Roh-, Hilfs- und Zwischenstoffen für die pharmazeutische und kosmetische Industrie, bei dem die sortengetrennten Produkte nach deren allfälligem Auflockern und/oder Sieben in produktabhängigen Schichtdicken in ein Prozessgebinde eingefüllt werden, wobei das Prozessgebinde identische Bebandlungsbedingungen für die gesamte Produktecharge gewährt mit dem Ziel der Erreichung einer homogenen Produktequalität, wonach das Prozessgebinde in eine Druckkammer eingebracht wird, in welcher der eigentliche Entkeimungsprozess abläuft, und es anschliessend aus der Druckkammer entnommen und das Produkt vor oder nach seiner Kühlung auf eine vorgegebene Temperatur aus dem Prozessgebinde entnommen wird, um dann allenfalls mittels einer mechanischen Nachbehandlung in seine ursprüngliche Form zurückgebracht zu werden, bevor es in das Auslieferungagebinde abgefüllt wird und/oder in die Weiterverarbeitung geht,
der eigentliche Entkeimungs- bzw. Entwesungsprozess programmgesteuert und homogen abläuft und eine Phase einer Konditionierung, in der die Druckkammer evakuiert wird eine Phase einer Entkeimung bzw. Entwesung , in der von Unterdruck startend Dampf in die Druckkammer eingelassen wird, sowie eine Phase einer Trocknung in der wieder annähernd ein Vakuum erzeugt wird, umfasst, **dadurch gekennzeichnet, dass** bei der Phase der Konditionierung die Druckkammer in mchroren Zyklen annähernd vakuumiert und wieder entvakuumiert wird, und dass die Phase des Entkeimens bzw. Entwesens nach Einlassen des Dampfes bei Umgebuzagsdruck und bei Temperaturen zwischen 70° und 120° C erfolgt.

2. Verfahren nach Anspruch 1, **dadurch gekennzeichnet, dass** die Produktoberfläche von natürlichen Trockenprodukten mit keiner unerwünschten Veränderung der sensorischen, physiologischen und chemischen Produkteigenschaften homogen und reproduzierbar entkeimt und entwest wird.

3. Verfahren nach Anspruch 1 oder 2, **dadurch gekennzeichnet, dass** die zu entkeimenden und entwesenden Produkte mittels einer Dosiervorrichtung in vorgegebenen Schichtdicken auf Tablare geschichtet und mit diesen in ein als Tablarträger ausgebildetes Prozessgebinde eingebracht werden.

4. Verfahren nach Anspruch 1 oder 2, **dadurch gekennzeichnet, dass** die zu behandelnden und entwesenden Produkte in produktabhängigen Schichtdicken in dampfdurchlässige Säcke eingebracht und diese in ein als Sackträger ausgebildetes Prozessgebinde eingebracht werden.

5. Verfahren nach einem der Ansprüche 1 bis 3, **dadurch gekennzeichnet, dass** das Produkt nach der Kühlung mittels eines geeigneten Werkzeuges (vorzugsweise eines Nibblers) in die ursprüngliche Form zurückgebracht wird.

6. Verfahren nach einem der Ansprüche 1 bis 5, **dadurch gekennzeichnet, dass** zur Durchführung des Entkeimungsprozesses Sattdampf eingesetzt wird.

7. Verfahren nach Anspruch 6, **dadurch gekennzeichnet, dass** der Sattdampf als pharmazeutischer Reindampf zugeführt wird.

8. Verfahren nach Anspruch 6 oder 7, **dadurch gekennzeichnet, dass** zum Trocknen die Druckkammer in mehreren Zyklen vakuumiert wird.

9. Verfahren nach einem der Ansprüche 6 bis 8, **dadurch** gekennzcichnct, dass das Entkeimen und Entwesen während einer Dauer von vorzugsweise 30 bis 300 s.

10. Verfahren nach einem der Ansprüche 1 bis 3 und 5 bis 9, **dadurch gekennzeichnet, dass** nach dem Verlassen der Druckkammer (7) bis und mit dem Abfüllen in die Auslieferungsgebinde die entkeimten und entwesten Produkte einen Reinraum nicht verlassen, mit Ausnahme der Produkte, die direkt in den Auslieferungsgebinden entkeimt und entwest werden.

11. Verfahren nach Anspruch 10, **dadurch gekennzeichnet, dass** die Luft in einem Reinraum bezüglich Temperatur, relativer Feuchtigkeit und mikrobieller Belastung produktabhängig konditioniert wird.

12. Verfahren nach einem der Ansprüche 1 bis 11, **dadurch gekennzeichnet, dass** eine passive Kühlung der Produkte durch Belassen derselbcn an der Umgebungsluft und/oder eine aktive Kühlung in einer Kühlkammer erfolgt.

13. Verfahren nach einem der Ansprüche 1 bis 12, **dadurch gekennzeichnet, dass** das Auslieferungsgebinde eine produktspezifische Luft- und Wasserdurchlässigkeit aufweist und keine mikrobielle Rekontaminierung zulässt.

14. Vorrichtung zum Durchführen des Verfahrens nach einem der Ansprüche 1 bis 3 und 5 bis 13, aufweisend eine Dosiervorrichtung (1), eine Einrichtung (2) zum Einbringen der Produkte in ein Prozessgebinde (3), eine an eine Vakuumpumpe, eine Dampfquelle und an eine Luftquelle angeschlossene Druckkammer (7), eine Kühlkammer (10), eine Entleerungseinrichtung (12) zum Entleeren der Prozessgebinde, Vorrichtungen für das Sieben, Mahlen, Brechen und Auflockern der Produkte, eine Verpackungseinrichtung (16), Kontrolleinrichtungen zur Bestimmung von Temperatur, Wassergehalt und Wasserverfügbarkeit in und um das Produkt sowie Steuerorgane zur Steuerung des Entkeimungs- und Entwesungsprozesses sowie der Kühlung.

15. Vorrichtung nach Anspruch 14, **dadurch gekennzeichnet, dass** das Prozessgebinde vorzugsweise als fahrbarer Tablarträger ausgebildet ist, in welchen mit Produkten beschichtete Tablare einbringbar sind.

16. Vorrichtung nach Anspruch 14, **dadurch gekennzeichnet, dass** das Prozessgebinde als fahrbarer Sackträger ausgebildet ist, der die gleichmässige Bedampfung der Sackoberflächen sicherstellt.

## Claims

1. Process for the sterilisation of bulk solid products, in particular foodstuffs and their raw materials, auxiliary products and semi-finished products, and also raw materials, adjuvant and intermediaries for the pharmaceutical and cosmetic industries, wherein the products, sorted according to type, and if necessary separated and/or sieved, are distributed in layers whose thickness depends on the product, in a process container, whereby the process container guarantees identical treatment conditions for the whole product batch with a view to achieving homogeneous product quality, whereupon the process container is inserted into a pressure chamber in which the actual sterilisation process takes place, whereafter the process container is removed from the pressure chamber and the product is taken out of the process container before or after it is cooled to a preset temperature, in order then to be restored to its original form by means of mechanical aftertreatment if necessary, before it is then filled into the outgoing delivery container and/or forwarded for further processing, that the actual sterilisation and disinfection process is carried out program-controlled and homogeneously and comprising a phase of a conditioning, in which the pressure chamber is placed under vacuum, a phase of a sterilisation respectively disinfection, with the inlet of steam in the pressure chamber starting from negative pressure, as well as a phase of a drying, in which again approximately a vacuum is generated, **characterised in that**
by the phase of the conditioning the pressure chamber is approximately vacuumised and again unvacuumised in several cycles, and that the phase of the sterilisation respectively disinfection after inlet of the steam take place by atmospheric pressure and temperatures between 70° and 120°C.

2. Process according to claim 1, **characterised in that** the product surface of natural dry products is sterilised and disinfected homogeneously and reproducibly with no unwanted alteration of the sensory, physiological and chemical product properties.

3. Process according to claim 1 or 2, **characterised in that** the products to be sterilised and disinfected are distributed in preset layer thicknesses on trays by means of a metering device and accommodated in a process container designed as a tray-holder.

4. Process according to claim 1 or 2, **characterised in that** the products to be treated and disinfected are distributed in steam permeable bags in product-dependent layer thicknesses and that these are then accommodated in a process container designed as a bag-holder.

5. Process according to one of the preceding claims 1 to 3, **characterised in that** the product is restored to its original form after cooling by means of a suitable tool (preferably a nibbler).

6. Process according to one of the preceding claims 1 to 5, **characterised in that** saturated steam is used to implement the sterilisation process.

7. Process according to claim 6, **characterised in that** the saturated steam is supplied as pharmaceutical pure steam.

8. Process according to claim 6 or 7, **characterised in that** the pressure chamber is placed under a vacuum in several cycles for the the drying.

9. Process according to one of the preceding claims 6 to 8, **characterised in that** the sterilisation and disinfection take place during a period of preferably 30 to 300 s.

10. Process according to one of the preceding claims 1 to 3 and 5 to 9, **characterised in that** after leaving the pressure chamber (7) up to and including filling into the outgoing delivery containers, the sterilised and disinfected products do not leave a clean room, with the exception of products, which are sterilised and disinfected directly in the outgoing delivery containers.

11. Process according to claim 10, **characterised in that** the air is conditioned in a clean room in terms of temperature, relative humidity and microbial load, depending on the product.

12. Process according to one of the preceding claims 1 to 11, **characterised in that** the products are cooled passively by leaving them in the ambient air and/or actively in a cooling chamber.

13. Process according to one of the preceding claims 1 to 12, **characterised in that** the outgoing delivery container displays a permeability to air and water specific to the product and allows no microbial recontamination.

14. Device for the execution of the process according to one of the claims 1 to 3 and 5 to 13, having a metering device (1), a device (2) for introducing the products into a process container (3), a pressure chamber (7) connected to a vacuum pump, a steam source and to an air source a cooling chamber (10), an emptying device (12) for emptying the process container, devices for sieving, grinding, breaking up and loosing up the products, a packing device (16), control devices for determining the temperature, water content and water availability in and around the product, together with control units for controlling the sterilisation and disinfection process and cooling.

15. Device according to claim 14, **characterised in that** the process container is preferably designed as a moveable tray-holder, into which trays with layers of the product on can be inserted.

16. Device according to claim 14, **characterised in that** the process container is designed as a moveable bag-holder, which ensures even vaporisation of the surfaces of the bag.

## Revendications

1. Procédé de stérilisation de produits solides fluides, notamment de produits alimentaires et de leurs matières premières, sous-produits et produits demi-finis, ainsi que de matières premières, secondaires et intermédiaires à destination de l'industrie pharmaceutique et cosmétique, procédé dans lequel des produits tamisés sont introduits après désagrégation et/ou tamisage dans un récipient de traitement avec des épaisseurs de couche dépendant du produit, le récipient de traitement étant maintenu dans des conditions de traitement identiques pour tout le lot de produit dans le but d'obtenir une qualité de produit homogène, puis le récipient de traitement est introduit dans une chambre de mise en pression dans laquelle s'effectue le processus de stérilisation proprement dit, puis l'emballage est retiré de la chambre de mise en pression et le produit est retiré du récipient de traitement avant ou après son refroidissement à une température prescrite afin qu'il revienne éventuellement dans sa forme d'origine par un traitement mécanique ultérieur avant sa mise dans le récipient de livraison et/ou l'exécution d'un traitement ultérieur, le processus de stérilisation respectivement de désinsectisation étant commandé par programme et se déroulant de façon homogène et comportant une phase de conditionnement dans laquelle la chambre de mise en pression est dépressurisée, une phase de stérilisation respectivement de désinsectisation dans laquelle on introduit de la vapeur dans la chambre de mise en pression en partant de l'état dépressurisé, et une phase de séchage dans laquelle on effectue une nouvelle dépressurisation approchée,
**caractérisé en ce que**, dans la phase de conditionnement, la chambre de mise en pression est à une dépressurisation approchée et est remise en pression en plusieurs cycles, et **en ce que** la phase de stérilisation respectivement de désinsectisation est effectuée après introduction de la vapeur à la pression ambiante et à des températures comprises entre 70° et 120°C.

2. Procédé selon la revendication 1, **caractérisé en ce que** la surface du produit est stérilisée et désinsectisée de façon homogène et reproductible par des produits secs naturels sans modification de façon indésirable des propriétés sensorielles, physiologiques et chimiques du produit.

3. Procédé selon la revendication 1 ou 2, **caractérisé en ce que** les produits à stériliser et désinsectiser sont placés sur des tablettes avec des épaisseurs de couche prescrites au moyen d'un dispositif de dosage et sont introduits avec ces tablettes dans un récipient de traitement conformé en support de tablette.

4. Procédé selon la revendication 1 ou 2, **caractérisé en ce que** les produits à traiter et désinsectiser sont introduits dans des sacs perméables à la vapeur avec une épaisseur de couche dépendant du produit et ces sacs sont introduits dans un récipient de traitement conformé en support de sac.

5. Procédé selon l'une des revendications 1 à 3, **caractérisé en ce que** le produit est ramené dans sa forme d'origine après refroidissement au moyen d'un outil approprié (avantageusement une grignoteuse).

6. Procédé selon l'une des revendications 1 à 5, **caractérisé en ce que** l'on utilise de la vapeur saturée pour effectuer le processus de stérilisation.

7. Procédé selon la revendication 6, **caractérisé en ce que** la vapeur saturée est amenée sous forme de vapeur pure du point de vue pharmaceutique.

8. Procédé selon la revendication 6 ou 7, **caractérisé en ce que** la dépressurisation est effectuée en plusieurs cycles pour sécher la chambre de mise en pression.

9. Procédé selon l'une des revendications 6 à 8, **caractérisé en ce que** la stérilisation et la désinsectisation sont effectuées avantageusement pendant une durée de 30 à 300 s.

10. Procédé selon l'une des revendications 1 à 3 et 5 à 9, **caractérisé en ce que**, après avoir quitté la chambre de mise en pression (7) et après avoir été introduits dans le récipient de livraison, les produits stérilisés et désinsectisés ne quittent pas une salle blanche à l'exception des produits qui sont stérilisés et désinsectisés directement dans les récipients de livraison.

11. Procédé selon la revendication 10, **caractérisé en ce que** l'air se trouvant dans une salle blanche est conditionné quant à sa température, son humidité relative et sa charge microbienne en fonction du produit.

12. Procédé selon l'une des revendications 1 à 11, **caractérisé en ce qu'**un refroidissement passif des produits est effectué par maintien de ces produits à l'air ambiant et un refroidissement actif est effectué dans une chambre de refroidissement.

13. Procédé selon l'une des revendications 1 à 12, **caractérisé en ce que** le récipient de livraison présente une perméabilité à l'air et à l'eau qui est spécifique du produit et ne permet aucune nouvelle contamination microbienne.

14. Dispositif destiné à mettre en oeuvre le procédé selon l'une des revendications 1 à 3 et 5 à 13, le dispositif comportant un dispositif de dosage (1), un dispositif (2) destiné à introduire les produits dans un récipient de traitement (3), une chambre de mise en pression (7) raccordée à une pompe à vide, une source de vapeur et une source d'air, une chambre de refroidissement (10), une chambre de vidange (12) destinée à vider le récipient de traitement, et des dispositifs de tamisage, de broyage, de concassage et de désagrégation des produits, un dispositif d'emballage (16), et des dispositifs de contrôle destinés à contrôler la température, la teneur en eau et la disponibilité de l'eau dans et autour du produit ainsi que des organes de commande destinés à commander le processus de stérilisation et de désinsectisation ainsi que le refroidissement.

15. Dispositif selon la revendication 14, **caractérisé en ce que** le récipient de traitement est avantageusement un support de tablettes transportable, dans lequel on peut introduire des tablettes recouvertes de produit.

16. Dispositif selon la revendication 14, **caractérisé en ce que** le récipient de traitement est un support de sacs transportable qui garantit la vaporisation uniforme de la surface des sacs.
